# EUROPÄISCHE PATENTSCHRIFT

(11) **EP 4 054 525 B1**
(45) Veröffentlichungstag und Bekanntmachung des Hinweises auf die Patenterteilung: **19.06.2024**
(21) Anmeldenummer: 20820325.7
(22) Anmeldetag: 21.11.2020
(51) Int. Cl.: A61K 9/00, A61K 9/06, A61K 9/48, A61K 9/51, A61K 9/70, A61K 35/00, A61K 47/46

(54) **BAKTERIOPHAGEN-BEREITSTELLUNGEN SOWIE BAKTERIOPHAGEN-APPLIKATIONSVORRICHTUNG**
PROVISION OF BACTERIOPHAGES IN VARIOUS DOSAGE FORMS AND BACTERIOPHAGE APPLICATION DEVICE
APPORT DE BACTÉRIOPHAGES SOUS DIVERSES FORMES POSOLOGIQUES ET DISPOSITIF D'APPLICATION DE BACTÉRIOPHAGES

(30) Priorität: 14.01.2020 DE 102020100725
(43) Veröffentlichungstag der Anmeldung: 14.09.2022
(73) Patentinhaber: PHATEC GMBH, 24116 Kiel (DE)
(72) Erfinder: JUNGHANS, Simon Frank, 24116 Kiel (DE); GROSS, Justus, 24235 Lutterbek (DE)
(74) Vertreter: Simmons & Simmons LLP (Munich)
(86) Internationale Anmeldenummer: PCT/DE2020/100988
(87) Internationale Veröffentlichungsnummer: WO 2021/143962

(56) Entgegenhaltungen:
- WO-A1-98/05344
- WO-A1-99/57304
- WO-A1-2006/047870
- CN-Y- 2 373 106
- CN-Y- 201 044 832
- US-A1- 2012 100 178
- US-A1- 2017 157 186

## Beschreibung

Die Erfindung betrifft eine intracorporale Bakteriophagen-Bereitstellung in Form eines Gels umfassend Bakteriophagen zur Verwendung in einem Verfahren zur Behandlung oder Prophylaxe von bakteriellen Infektionen, wobei das Gel steril ist und während einer Operation intracorporal appliziert wird.

Als Bakteriophagen, kurz auch BPH oder Phagen, bezeichnet man verschiedene Gruppen von Viren, die auf Bakterien als Wirtszellen spezialisiert sind, d.h. welche Bakterien spezifisch befallen. Damit wird der Wirt, beispielsweise ein Säugetier und insbesondere ein Mensch, nicht befallen. Eine Infektion von Bakterien oder anderen pathogenen Mikroorganismen durch virulente Phagen führt zum lytischen Zyklus und damit letztendlich zur Lyse und Zerstörung der Bakterien. Endotoxine sind Bakterientoxine, die im Gegensatz zu den Exotoxinen nicht von lebenden Bakterien ausgeschieden werden, sondern erst durch Autolyse freigesetzt werden.

Phagen haben in Medizin, Tiermedizin Biologie, Agrarwissenschaften, Lebensmittelverarbeitung, vor allem im Bereich der Gentechnologie, ein breites Anwendungsspektrum gefunden. So verwendet man Phagen in der Medizin aufgrund ihrer Wirtsspezifität zur Bestimmung von bakteriellen Erregern. Die Anwendung von Phagen zur Therapie bakterieller Infektionen entdeckte Felix d'Herelle lange vor Entdeckung des Penicillins und der Antibiotika. Später wurde die Phagentherapie jedoch mit der Einführung der Chemotherapie per Antibiotika als unpraktisch erachtet und geriet in Vergessenheit. Aufgrund der immer häufiger auftretenden multiplen Antibiotikaresistenzen wird derzeit wieder intensiv an der Anwendung von Bakteriophagen als Antibiotika-Ersatz in der Humanmedizin geforscht.

Eine Reihe von Publikation greifen diesen Stand der Technik auf und beschäftigen sich mit dem Stand der Phagentherapien allgemein und den regionalen Stati der medizinischen Zulassungen von Medikamenten, die auf Phagen basieren.

Aus der Druckschrift US 2004/0063 189 A1 ist eine Methode zur Identifikation und Bekämpfung des Bacillus Anthrax auf Oberflächen durch Phagen bekannt.

Weiter ist aus der Publikation Qadir et al. "Phage therapy: progress in pharmacokinetics", Braz. J. Pharm. Sei. 2018; 54(1):e17093, S.1-9 eine Ausführung zum allgemeinen Stand von Phagentherapien und den regionalen Stati der medizinischen Zulassungen von Medikamenten, die auf Phagen basieren, bekannt.

Aus der Druckschrift WO 2006/047870 A1 sind Bakteriophagen-Zusammensetzungen und Verfahren zu deren Herstellung bekannt.

Bakteriophagen können aus der Natur gewonnen werden. Dazu werden Wasserproben, Blutproben, Abstriche, menschliche oder tierische Sekrete, oder andere Proben genommen und auf Nährplatten ausgestrichen. Durch Bebrüten dieser Platten (36 °C - 37 °C für 24 h) findet man, indiziert durch Lyse-Höfe, vorhandene Bakteriophagen. Durch Detektion der vorhandenen Prokaryoten kann eine erste Aussage getroffen werden, gegen welches Bakterium der gefundene Phage lytische aktiv ist.

Zur Aufreinigung wird der Plaque im Bakterienrasen ausgestochen und in einem Schnappdeckel-Gefäß mit flüssiger Nährlösung für mindestens 10 Minuten gevortext. Die flüssige Nährlösung wird entnommen, steril-filtriert und auf einer vorher mit dem entsprechenden Keim beimpften Nährmedium-Platte aufgetragen und wieder für 24 h bei 36 °C - 37 °C bebrütet. Eine Plaque wird wieder ausgestochen und wie beschrieben aufbereitet. Diesen Zyklus sollte man mindestens 5 Mal wiederholen, um sicher zu sein, dass die vorhandenen, isolierten Phagen nur Klone eines Phagen sind.

Zur Generierung größerer Mengen Phagenklone eines Phagen wird die steril filtrierte Phagenlösung aus dem Schnappdeckel-Gefäß nach mindestens 5-facher Aufreinigung auf einer beimpften Platte aufgebracht und erneut wie beschrieben bebrütet. Dann wird auf die Platte ein Extraktionspuffer gegeben, welcher auf dem Nährboden für 30 Minuten durch eine Schüttel-Apparatur bewegt wird. Der Extraktionspuffer wird entnommen und steril-filtriert, dabei erhält man eine sterile Phagen-Lösung.

Eine Bakteriophagen-Lösung kann über Zugabe von Stabilisatoren, wie bspw.: CaCl2 stabilisiert und über Substanzen zur pH-Einstellung wie bspw.: HCl, CH3COOH, CH3COO- physiologisch eingestellt werden. Der weitere Zusatz von Konservierungsmitteln kann dadurch indiziert sein, dass die Primärverpackung nicht vor dem Eindringen von Mikroorganismen schützt (ggf. bei nicht sterilen Produkten). Hier werden Konservierungsmittel wie bspw.: Kaliumsorbat eingesetzt.

Antimikrobielle Resistenzen entwickeln sich weltweit zum ernsten Problem für das Gesundheitswesen. Über Jahrzehnte hinweg wurde nur unzureichend an der Erforschung grundlegend neuer Antibiotika gearbeitet, folglich erreichten nur wenige Präparate den Markt. Der Druck ist seither enorm gewachsen, neue wirksame Konzepte zur Reduktion von Infektionen durch Problemerreger zu implementieren. Von politischer Seite wurde diese Dringlichkeit erkannt und umfangreiche Förderprogramme wurden sowohl national als auch international ins Leben gerufen. Eine tragende Säule vieler öffentlich finanzierter Maßnahmen ist die Erforschung und Entwicklung von Therapeutika, deren Wirkungen auf neuen Mechanismen beruhen und/oder die Bildung von Resistenzen minimieren.

In medizinischen Bereichen ist die Infektion eines Fremdkörpers mit einer erhöhten Komplikations- und Mortalitätsrate verbunden, diesbezüglich ist die derzeitige und prospektive Antibiotikum- Therapie ausgereizt.

Der dringende Bedarf an Antibiotika-Alternativen ist die Forderung für die diesseitige Erfindung.

Probleme ergeben sich durch die geringe Stabilität von Phagen im Körper, da sie in recht kurzer Zeit durch Fresszellen als Fremdkörper beseitigt werden.

Der vorliegenden Erfindung liegt die Aufgabe zugrunde Bakteriophagen-Bereitstellungen, auch als Bakteriophagen-Depots bezeichnet, aufzuzeigen, die durch technische Vorrichtungen applizierbar sind sowie entsprechende Applikationsvorrichtungen hierzu aufzuzeigen, um entsprechende Bakteriophagen-Bereitstellungen zeitlich und örtlich dosierbar zu applizieren, um auf dieser Basis ein präventives Antibiotikum mit Breitbandwirkung ohne Nebenwirkungen einfach applizierbar bereitzustellen.

Insbesondere ist es Ziel, Bakteriophagen in unterschiedlichem Aggregatszustand und galenischer Zusammensetzung biologisch aktiv als Infektionsprophylaxe oder als Infektionstherapie an Fremdkörper und an homo- sowie xenogenes Gewebe zu applizieren.

Gelöst wird diese Aufgabe bzw. Aufgaben durch die erfindungsgemäße intracorporale Bakteriophagen-Bereitstellung in Form eines Gels umfassend Bakteriophagen zur Verwendung in einem Verfahren zur Behandlung oder Prophylaxe von bakteriellen Infektionen, wobei das Gel steril ist und während einer Operation intracorporal appliziert wird.

Eine Zwei-Spritzen-Bakteriophagen-Bereitstellung kennzeichnet sich dadurch aus, dass eine erste Spritze mit einer Bakteriophagen-Lösung vorbereitet ist und eine zweite Spritze mit einem Gel vorbereitet ist, wobei diese zwei Spritzen miteinander, insbesondere über einen Konnektor, verbindbar sind, wobei eine Vermischung des Gels mit der Bakteriophagen-Lösung möglich ist und diese dann in einer Spritze zur Applikation vorliegt. Hierbei können die Gele und auch die Bakteriophagen-Lösungen entsprechend an die Bedürfnisse angepasst werden. Insbesondere können unterschiedliche Gele und/oder unterschiedliche Bakteriophagen-Lösungen zur unterschiedlichen Kombination miteinander vorgehalten und entsprechend miteinander zu einem individuellen Bakteriophagen-Lösung-Gel vermischt werden.

Im Folgenden werden die zuvor genannten Ausführungen der Erfindung detailliert noch beschrieben:
Ein Bakteriophagen-Depot oder Phagen-Bereitstellung bzw. Depot ist eine Einheit, in der zumindest ein Bakteriophage oder Phage als eine Phagen-Applikation bereitgestellt wird, die die Stabilität nach Einbringung in einen Körper zeitlich definierbar aufrechterhält.

Es wurde insbesondere erkannt, dass der Bakteriophage sich sowohl für eine Behandlung als auch für eine Prophylaxe von bakteriellen Erkrankungen oder Entzündungen oder Pilzerkrankungen eignet, insbesondere aufgrund der sehr geringen damit verbundenen Nebenwirkungen. Aufgrund des Wirkmechanismus, der eine Vervielfältigung der aktiven Bakteriophagen erst bei Auftreten einer Infektion des Wirtes mit einem entsprechenden Bakterium oder Pilz beinhaltet, ist es möglich, prophylaktisch geringe Mengen an Bakteriophagen und Endotoxinen zu verabreichen, die den Wirtsorganismus nicht schädigen, die aber bei Auftreten von zu bekämpfenden Bakterien oder Pilzen in kurzer Zeit vervielfacht werden. Im Allgemeinen ist bei akuten Infektionen die Verabreichung hoher Dosen an Bakteriophagen möglich, da diese den pathogenen Mikroorganismus selektiv lysieren, ohne den Wirtsorganismus, z. B. einen Säuger, zu schädigen.

Im Folgenden werden beispielhaft technische Umsetzungen der Bakteriophagen-Applikation zur Verwendung gemäß der beanspruchten Erfindung dargestellt. Das Ziel der dargestellten Bakteriophagen-Applikation ist die Einbringung von therapeutisch aktiven Bakteriophagen zur Prävention und / oder Therapie und / oder Minimierung einer bakteriell bedingten Infektion, wobei es hier nicht um das therapeutische Verfahren geht, sondern vielmehr um die dazu notwendigen Produkte / Applikationen.

### Intracorporale Applikation:

### Steriles Bakteriophagen-Gel (freisetzungsmoduliert)

Das sterile Bakteriophagen-Gel ist überall intracorporal applizierbar. Die Herstellung erfolgt aus der entsprechenden Bakteriophagen-Lösung heraus:
Ein Gel wird mittels eines Gelbildners (bspw.: HPMC, ...), ohne den Wasseranteil, den später die Bakteriophagen-Lösung stellt, hergestellt und sterilisiert. Es können adhäsive Substanzen zugemischt werden, die die Adhäsion an unterschiedlichen Materialen (PTFE, Keramik, Dacron, Titan, ...) verbessern. Die Bakteriophagen-Lösung wird unter sterilen Bedingungen steril-filtriert und in einer Spritze steril vorgehalten. Das sterilisierte Gel wird unter sterilen Bedingungen auch in einer Spritze vorgehalten. Zur besseren Lagerung werden die beiden Komponenten durch eine Zwei-Spritzen-Technik durch ein operierendes Team erst vor Applikation vermischt.

Die Eigenschaften des resultierenden Gels und damit auch die Bakteriophagen- Freisetzungsrate aus dem selbigen werden über die benutze Menge an Gelbildner definiert.

Aus diesem Grund sollen unterschiedliche Gelgrundlagen in Spritzen vorgehalten werden. Alle Bakteriophagen-Lösungen, welche sich in ihrer Zusammensetzung der Bakteriophagen unterscheiden, sind mit allen Gelgrundlagen durch die Zwei-Spritzen-Technik kombinierbar.

Ein Operateur kann also intra-operativ entscheiden, welche Viskosität / Freisetzung und welche Bakteriophagen-Zusammensetzung er applizieren möchte. Dabei sind weniger viskose Gele schnell freisetzend und hoch-visköse Bakteriophagen-Gele setzen über einen längeren Zeitraum frei. Die einzelnen Spritzen (Gelgrundlage und Bakteriophagen-Lösung) sind jeweils steril verpackt und werden auf Verlangen vom unsterilen OP-Personal steril angereicht. Das sterile OP-Personal mischt die Komponenten mit Hilfe der Zwei-Spritzen-Technik über einen vorgegebenen Zeitraum. Das Bakteriophagen-Gel ist jetzt zur Applikation bereit.

Weitere Herstellungsvarianten:
- über maschinelle Produktion werden Bakteriophagen-Lösungen mit einem ersten Teil des Gels vermischt und steril-filtriert. Die Modulation dieses Grundgels erfolgt durch entsprechendes Zumischen des Gelbildners unter sterilen Bedingungen in einem nachgeschalteten Produktionsschritt;
- maschinelle, sterile Abfüllung von Bakteriophagen-Lösung und Grundgel. Das Grundgel wird anschließend im Endbehältnis sterilisiert. Ein Zusammenbringen ist flexibel gleich dem oben initial beschriebenen, händischen Verfahren.

Weitere Möglichkeiten steriler Bakteriophagen-Gele:
A. die Bakteriophagen sind in einer Hydrogel-Matrix homogen verteilt eingebettet. Die Freisetzung aus dem Hydrogel wird durch den Anteil an Hydrogelbildner im Verhältnis zum Wasseranteil bestimmt. Das Produkt interagiert nicht mit Haut- oder Schleimhautzellen, ist gegen den menschlichen Organismus inert und kann intracorporal zum Einsatz kommen; mögliche Gelbildner sind Carbomere, Celluloseether, Gelatine, Alginate, Betonid, hochdisperses Siliciumdioxid.
B. Die Phagen sind in einer lipophilen Gel-Matrix eingebettet. Die Freisetzung aus dem Hydrogel wird durch den Anteil an Lipophilgelbildner im Verhältnis zum Wasseranteil bestimmt. Das Produkt ist über den antibakteriellen Mechanismus hinaus stark rückfettend und unterstützt den natürlichen Wundheilungsprozess als Ergänzung zur antibakteriellen Therapie / Prävention.
   Mögliche lipophile Gelbildner sind hochdisperses Siliciumdioxid, Aluminium seifen, Zinkseifen, insbesondere jeweils mit entsprechender, lipophiler Grundlage, wie Mineralöl oder flüssige Triglyceride.
C. Die Bakteriophagen sind in einer amphiphilen Gel-Matrix eingebettet. Die Freisetzung aus dem Hydrogel wird durch den Anteil an amphiphilem Gelbildner im Verhältnis zum Wasseranteil bestimmt.

Alle Gele können auch über die Spritze minimalinvasiv eingebracht werden. Auch eine percutane Applikation, bspw.: in einen Abszess, ist also möglich.

Im Folgenden werden beispielhaft technische Umsetzungen der Bakteriophagen-Applikation zur Verwendung gemäß der beanspruchten Erfindung dargestellt. Das Ziel der dargestellten Bakteriophagen-Applikation ist die Einbringung von therapeutisch aktiven Bakteriophagen zur Prävention und / oder Minimierung einer bakteriell bedingten Infektion.

Es werden Ausführungsbeispiele der Erfindung anhand der beiliegenden Zeichnung in der **Figurenbeschreibung** detailliert beschrieben und zusätzliche nachfolgend auch weitere andere Ausführungsvarianten aufgezeigt, die nicht figürlich dargestellt sind. Diese Ausführungen sollen die Erfindung erläutern und sollen nicht beschränkend zu werten sein:
Es zeigt:

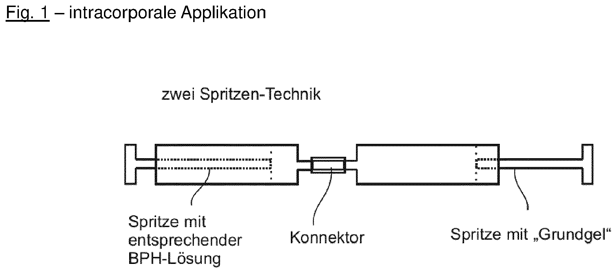

Fig. 1 eine schematische Darstellung eines Ausführungsbeispiels einer intracorporale Bakteriophagen-Applikation;

In **Fig. 1** ist eine schematische Darstellung eines Ausführungsbeispiels einer intracorporale Bakteriophagen-Applikation dargestellt, wobei aus einer Bakteriophagen-Lösung ein steriles Bakteriophagen-Gel hergestellt wird und über Bakteriophagen-Applikations-Vorrichtungen überall intracorporal applizierbar ist.

Bei der Herstellung wird ein Gel mittels eines Gelbildners, beispielsweise HPMC oder dgl. , ohne den Wasseranteil der späteren Bakteriophagen-Lösung, hergestellt und sterilisiert. Es können dabei adhäsive Substanzen zugemischt werden, die die Adhäsion an unterschiedlichen Materialen (PTFE, Keramik, Dacron, Titan, Zinkoxid...) verbessern.

Die Bakteriophagen-Lösung wird unter sterilen Bedingungen steril-filtriert und z.B. in einer Spritze steril vorgehalten. Das sterilisierte Gel wird unter sterilen Bedingungen z.B. in einer Spritze vorgehalten. Zur besseren Lagerung werden die beiden Komponenten durch eine Zwei- Spritzen-Technik zeitlich erst unmittelbar vor einer Applikation vermischt.

Die Eigenschaften des resultierenden Gels und damit auch die Bakteriophagen- Freisetzungsrate aus dem selbigen werden über die benutze Menge an Gelbildner definiert.

Aus diesem Grund werden unterschiedliche Gelgrundlagen z.B. in Spritzen vorgehalten. Alle Bakteriophagen-Lösungen, welche sich in ihrer Zusammensetzung der Bakteriophagen unterscheiden, sind mit allen Gelgrundlagen damit durch die Zwei-Spritzen-Technik kombinierbar.

Weitere Möglichkeiten der Bereitstellung sind z.B. eine schrittweise Vorgehensweise, bei der z.B. über maschinelle Produktion Bakteriophagen-Lösungen mit einem ersten Teil des Gels vermischt und steril-filtriert werden und die Modulation dieses Grundgels durch entsprechenden Zumischen des Gelbildners unter sterilen Bedingungen in einem nachgeschalteten Produktionsschritt erfolgt. Weiter ist es möglich, die maschinelle, sterile Abfüllung von BPH-Lösung und Grundgel durchzuführen und das Grundgel anschließend im Endbehältnis zu sterilisieren. Ein Zusammenbringen kann dann flexibel erfolgen.

Weitere Möglichkeiten sterile Bakteriophagen-Gele bereitzustellen, sind z.B. die Phagen in einer Hydrogel-Matrix homogen verteilt einzubetten. Die Freisetzung aus dem Hydrogel wird dabei durch den Anteil an Hydrogelbildner im Verhältnis zum Wasseranteil bestimmt. Das Produkt interagiert nicht mit Haut- oder Schleimhautzellen, ist gegen den menschlichen Organismus inert und kann intracorporal zum Einsatz kommen, oder die Phagen in einer lipophilen Gel-Matrix einzubetten. Die Freisetzung aus dem Hydrogel wird dabei durch den Anteil an Lipophilgelbildner im Verhältnis zum Wasseranteil bestimmt. Das Produkt ist über den antibakteriellen Mechanismus hinaus stark rückfettend und unterstützt den natürlichen Wundheilungsprozess als Ergänzung zur antibakteriellen Therapie / Prävention. Die Phagen können in einer amphiphilen Gel-Matrix eingebettet sein. Die Freisetzung aus dem Hydrogel wird dabei durch den Anteil an amphiphilem Gelbildners im Verhältnis zum Wasseranteil bestimmt.

Alle Gele können auch über die Spritze minimalinvasiv eingebracht werden. Auch eine percutane Applikation, bspw. in einen Abszess, ist nunmehr erstmalig möglich, um diesen zu behandeln.

Es kann unmittelbar vor der Applikation die Viskosität, Freisetzungsrate und BPH-Zusammensetzung eingestellt werden. Dabei sind weniger viskose Gele schnell freisetzend und hoch-visköse BPH-Gele setzen über einen längeren Zeitraum frei.

Ein besonders hervorzuhebendes Anwendungsbeispiel für eine gezielte Applikation von Phagen ist die Prothetik.

Protheseninfektionen stellen auch heute noch eine der gravierendsten Komplikationen der rekonstruktiven Chirurgie dar. Protheseninfektion, insbesondere wenn beispielsweise die Aorta betroffen ist, haben oft einen letalen Verlauf; gerade dieses chirurgische Fachgebiet stellt sich nicht nur als hochkompliziert, sondern auch aufgrund der hohen Anzahl verwendeter Kunststoffprothesen als besonders komplikationsträchtig dar.

## Patentansprüche

1. Gel umfassend Bakteriophagen zur Verwendung in einem Verfahren zur Behandlung oder Prophylaxe von bakteriellen Infektionen, wobei das Gel steril ist und während einer Operation intracorporal appliziert wird.

## Claims

1. A gel comprising bacteriophages for use in a method for the treatment or prophylaxis of bacterial infections, wherein the gel is sterile and is applied intracorporeally during an operation.

## Revendications

1. Gel comprenant des bactériophages destiné à être utilisé dans une méthode de traitement ou de prophylaxie d'infections bactériennes, ledit gel étant stérile et étant appliqué par voie intracorporelle pendant une opération.
